# EUROPEAN PATENT APPLICATION

(11) **EP 4 527 304 A1**
(43) Date of publication of application: **26.03.2025**
(21) Application number: 23198794.2
(22) Date of filing: 21.09.2023
(51) Int. Cl.: A61B 6/10, A61B 6/00

(54) **C-ARM SYSTEM**

(71) Applicant: Koninklijke Philips N.V., 5656 AG Eindhoven (NL)
(72) Inventor: JALGAONKAR, Mahesh, Eindhoven (NL); KARULE, Mahendra Vasantrao, Eindhoven (NL)
(74) Representative: Philips Intellectual Property & Standards

(57) **Abstract**

Proposed concepts thus aim to provide schemes, solutions, concept, designs, methods and systems pertaining to a C-arm system comprising at least one electro-magnetic (EM) brake for locking movement of a C-arm when not supplied with power. In particular, embodiments aim to provide a power unit for a C-arm system comprising at least one electro-magnetic (EM) brake for locking movement of a C-arm when not supplied with power which includes a power storage unit, an interface configured to electrically connect to the EM brake(s) of the C-arm system, such that power can be supplied from the power storage unit to the brake(s), as well as a switch able to essentially turn power from the power storage unit on and off when desired.

## Description

### FIELD OF THE INVENTION

This invention relates to the field of C-arm systems, and more specifically, to the field of power units for C-arm system.

### BACKGROUND OF THE INVENTION

Mobile (2D/3D) C-arm systems may comprise electro-magnetic (EM) brakes providing mechanical resistance when they are electrically powered or unpowered. Such resistance is automatically released during the use of the C-arm, and suddenly operated to stop the movement of the C-arm at a predetermined stop position or when an obstacle is met or a danger is detected. Such EM brakes are therefore very useful to get better control and greater security during use of the C-arm, and to keep the C-arm substantially immovable when the C-arm is not being used (i.e., turned off or not connected to power).

### SUMMARY OF THE INVENTION

The invention is defined by the claims.

According to examples in accordance with an aspect of the invention, there is provided a power unit for a C-arm system comprising at least one electro-magnetic (EM) brake for locking movement of a C-arm when not supplied with power.

The power unit comprises: a power storage unit; an interface configured to electrically connect to the at least one EM brake of the C-arm system for supplying power to the at least one EM brake when the interface is electrically powered; and a switch operable between a first configuration and a second configuration, wherein in the first configuration, the switch is configured to permit power supply from the power storage unit to the interface, and in the second configuration, the switch is configured to not permit power supply from the power storage unit to the interface.

Proposed concepts thus aim to provide schemes, solutions, concept, designs, methods and systems pertaining to a power unit for a C-arm system comprising at least one electro-magnetic (EM) brake for locking movement of a C-arm when not supplied with power. In particular, embodiments aim to provide a power unit for a C-arm system comprising at least one electro-magnetic (EM) brake for locking movement of a C-arm when not supplied with power which includes a power storage unit, an interface configured to electrically connect to the EM brake(s) of the C-arm system, such that power can be supplied from the power storage unit to the brake(s), as well as a switch able to essentially turn the power from the power storage unit on and off.

In other words, it is proposed that by providing a power unit which includes its own power storage unit, power can be supplied to the EM brake(s) of a C-arm system even when the C-arm system is not supplied with power from another source, e.g., mains power. For example, during a power cut and/or during transportation, power can still be supplied to the EM brake(s) of the C-arm system via the power unit. In this way, the EM brake(s) can be disengaged, and the C-arm can be maneuvered.

Further, by providing a switch, power can be supplied to the EM brake(s) of the C-arm system only when it is needed. This reduces energy drainage from the power storage unit when it is not required, for instance, when the C-arm is already connected to an external source of power, or when disconnected but the C-arm does not need to be moved.

It is not possible to manually position the C-arm when the C-arm system is not connected to mains power. This is because, due to fail-safe mechanisms, the EM brake(s) can only be switched off when powered. Therefore, it is currently not possible (or requires a lot of force) to position the C-arm when not connected to mains power, e.g., during transport through the hospital or during a power cut.

In a simple example, the EM brake(s) of a C-arm activate when mains power to the C-arm fails. In this situation, movement of the C-arm becomes very difficult - and if this occurs during an operation/surgery, this can potentially be dangerous. With the addition of the power unit, however, including an interface configured to electrically connect to the EM brake(s) of the C-arm system, power can be supplied from the power unit's power storage unit to the EM brake(s), such that they can be disengaged, and the C-arm moved.

In another example, during transportation, if there is a need to set the C-arm to a particular position to allow it to pass through a door, typically the C-arm would need to be connected to mains power to be powered up fully. However, with the use of the present invention, there is no need to connect the C-arm to mains power and/or power up the entirety of the C-arm. Instead, merely by activating a switch (or pressing a button) power can be supplied from a power storage unit to the EM brake(s) of the C-arm (for a particular duration, for example, two minutes) such that the position of the C-arm can be more easily and/or efficiently adjusted.

The manual override force required to move a C-arm when the EM brake(s) are active is high and will also reduce the working life of the EM brake(s). When manually overriding the EM brake(s), i.e., re-positioning the C-arm even with the EM brakes active, the EM brake(s) undergo wear and damage. This invention thus facilitates the potential lengthening of the EM brake(s)' lifespan by eliminating any need to manually override the EM brake(s). Further, during serviceability, the invention can be used to more easily identify faults in EM brake(s) and/or their respective drives or motors.

Ultimately, an improved power unit for a C-arm system comprising at least one electro-magnetic (EM) brake for locking movement of a C-arm when not supplied with power is provided.

In some embodiments, the C-arm system may comprise at least one motor to drive a movement of the C-arm, a radiation source and/or a detector; and wherein the interface may be configured to not electrically connect with any motor, radiation source, or detector. In this way, power from the power storage unit is reserved for the EM brake(s) of the C-arm system and not wasted on any motors, radiation sources or detectors, even if the C-arm system comprises these components.

In some embodiments, the interface may be configured to electrically connect exclusively to at least one EM brake of the C-arm system. In this way, power from the power storage unit is directed exclusively to the EM brake(s) such that energy usage for the purpose of re-positioning the C-arm system is made more efficient. For example, by not also providing power to the X-ray system of the C-arm system, when it is only the EM brake(s) which require power for the repositioning of the C-arm, power usage is made more efficient.

In some embodiments, the C-arm system may comprise at least one motor to drive a movement of the C-arm, and wherein the interface may be configured to electrically connect exclusively to the at least one EM brake and at least one motor of the C-arm system. In this way, power can also be provided to motors of the C-arm system such that positioning of the C-arm can be made easier, while still not providing power to other extraneous components of the C-arm system, such as, for example, its X-ray system.

In some embodiments, the power unit may further comprise a charging unit configured to facilitate charging of the power storage unit within the power unit. In this way, the power storage unit does not need to be removed from the power unit in order for the power storage unit to be recharged and/or for the power unit to be supplied with more power.

In some embodiments, the power storage unit may be removable. In this way, an empty power storage unit can be swapped out for a full power storage unit, and/or the power storage unit can be removed so that it can be charged and placed back in, and/or the power storage unit can be easily replaced if it is faulty.

In some embodiments, the power unit may further comprise a timer configured to put the switch in the second configuration after a predetermined time period of the switch being in the first configuration. In this way, for instance, power can automatically stop being supplied from the power storage unit to the EM brake(s) after, for example, two minutes. This therefore prevents energy waste in cases in which, for example, a user forgets to turn the power supply from the power storage unit back off after they have adjusted the position of the C-arm. For example, two minutes should be long enough for re-positioning of the C-arm to any position, and if it is not, the user may subsequently put the switch back in the first configuration again.

In some embodiments, the timer may be programmable by a user or by the C-arm system for adjusting the predetermined time period. In this way, the timer can be adjusted for increased efficacy and/or efficiency.

In some embodiments, the switch may be configured not to be in the first configuration if the at least one EM brake of the C-arm is already being supplied with power from another power source. In this way, the supply of power from the power storage unit can be prevented when it is not needed. For instance, if the C-arm is already connected to mains power, then there is no need for the EM brake(s) to also be supplied with power from the power storage unit. Energy efficiency can thus be increased.

In some embodiments, the switch may be configured not to be in the first configuration if the C-arm system is in use. In this way, supply of power from the power storage unit can be prevented when it is not needed.

In some embodiments, the switch may comprise a physical switch and/or a button. In this way, a user can easily interact with the switch and easily supply power from the power storage unit to the EM brake(s) of the C-arm system when desired.

In some embodiments, the switch may comprise an indicator configured to indicate to a user whether the switch is in the first configuration or the second configuration. In this way, a user can be supplied with an indication of whether the power unit is currently supplying power to the EM brake(s) of the C-arm system, i.e., which configuration the switch is in.

In some embodiments, the C-arm system may comprise a plurality of EM brakes; the interface may be configured to independently electrically connect to individual EM brakes of the C-arm system for supplying power to each EM brake respectively; and the switch may comprise a plurality of sub-switches operable between first and second sub-configurations respectively, wherein in a first sub-configuration, each sub-switch is configured to facilitate electrical communication between the power storage unit and an individual EM brake of the C-arm system respectively, and in a second sub-configuration, each sub-switch is configured to not permit power supply from the power storage unit and an individual EM brake of the C-arm system respectively.

In this way, specific EM brakes of the C-arm system can be targeted for deactivation/activation. For instance, movement of the C-arm may only be desired in one particular axis, and therefore, power only needs to be supplied to one EM brake (set) for the desired movement to be achieved. By not automatically supplying power to all EM brakes, but rather allowing users to specify which brakes they want to supply power to, energy efficiency can thus be improved.

In some embodiments, two or more sub-switches may be operated simultaneously. In this way, a user can turn multiple EM brakes on or off simultaneously, for instance, from a single command or button press.

In some embodiments, the power unit may be configured to connect to a key-panel of the C-arm system. In this way, the key-panel can essentially act as a physical switch and/or button or set of physical switches and/or buttons. This therefore makes efficient use of the physical hardware available.

In some embodiments, the switch may be operable between the first configuration and the second configuration after a press of the physical switch and/or button of at least five seconds. In this way, false operations of the switch/button can be reduced and thus energy wastage prevented.

In some embodiments, the indicator may comprise a light source, wherein the light source is configured to emit light in the first configuration and to not emit light in the second configuration.

In some embodiments, the C-arm of the C-arm system may be configured to translate and/or rotate in at least five axes. This is a particularly useful number of axes of movement for a C-arm system.

Thus, there may be proposed concepts for a power unit for a C-arm system comprising at least one electro-magnetic (EM) brake for locking movement of a C-arm when not supplied with power, and this may be done by including a power storage unit, an interface, and a switch in the power unit.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a simplified block diagram of a power unit for a C-arm system comprising at least one electro-magnetic (EM) brake for locking movement of a C-arm when not supplied with power according to a proposed embodiment;
Fig. 2 is a block diagram of a power unit for a C-arm system comprising at least one electro-magnetic (EM) brake for locking movement of a C-arm when not supplied with power according to a proposed embodiment;
Fig. 3 is a block diagram of a power unit for a C-arm system comprising at least one electro-magnetic (EM) brake for locking movement of a C-arm when not supplied with power according to a proposed embodiment;
Fig. 4 is an illustration of atypical C-arm system and its axes of motion;
Fig. 5 is a circuit diagram of a C-arm system comprising a power unit according to a proposed embodiment; and
Fig. 6 illustrates an example of a computer within which one or more parts of an embodiment may be employed.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Implementations in accordance with the present disclosure relate to various techniques, methods, schemes and/or solutions pertaining to a C-arm system comprising at least one electro-magnetic (EM) brake for locking movement of a C-arm when not supplied with power. According to proposed concepts, a number of possible solutions may be implemented separately or jointly. That is, although these possible solutions may be described below separately, two or more of these possible solutions may be implemented in one combination or another.

Embodiments of the invention aim to provide a power unit for a C-arm system comprising at least one EM brake for locking movement of a C-arm when not supplied with power. This can be achieved by including a power storage unit, an interface, and a switch in the power unit.

Proposed concepts thus aim to provide schemes, solutions, concept, designs, methods and systems pertaining to a power unit for a C-arm system comprising at least one electro-magnetic (EM) brake for locking movement of a C-arm when not supplied with power. In particular, embodiments aim to provide a power unit for a C-arm system comprising at least one EM brake for locking movement of a C-arm when not supplied with power which includes a power storage unit, an interface configured to electrically connect to the EM brake(s) of the C-arm system, such that power can be supplied from the power storage unit to the brake(s), as well as a switch able to essentially turn the power from the power storage unit on and off when desired.

In other words, it is proposed that by providing a power unit which includes its own power storage unit, power can be supplied to the EM brake(s) of a C-arm system even when the C-arm system is not supplied with power from another source, e.g., mains power. For example, during a power cut and/or during transportation, power can still be supplied to the EM brake(s) of the C-arm system via the power unit. In this way, the EM brake(s) can be disengaged and the C-arm can still be maneuvered.

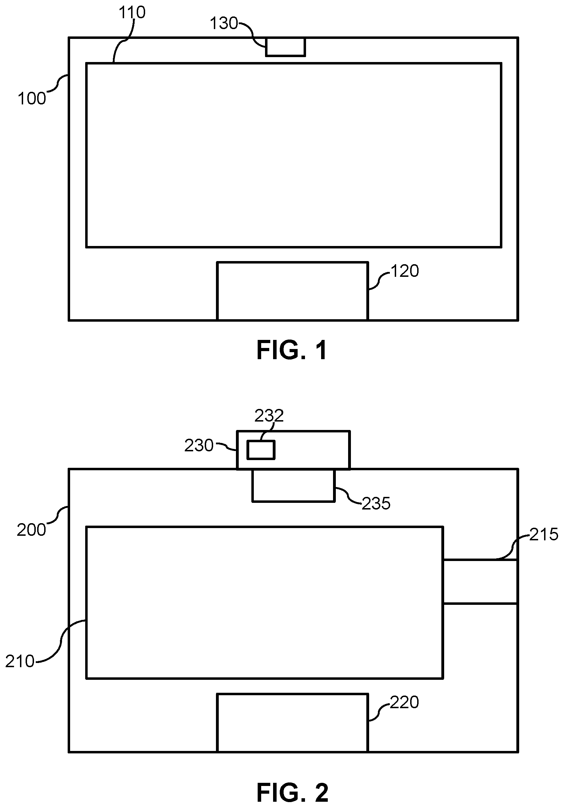

Referring now to Fig. 1, there is depicted a simplified block diagram of a power unit 100 for a C-arm system comprising at least one electro-magnetic (EM) brake for locking movement of a C-arm when not supplied with power according to a proposed embodiment.

The power unit 100 comprises a power storage unit 110, an interface 120, and a switch 130.

The power storage unit 110 can be understood as a battery or any other unit/component capable of storing power/energy. In this embodiment, the power storage unit 110 is removable. In this way, an empty power storage unit can be swapped out for a full power storage unit, and/or the power storage unit can be removed so that it can be charged and placed back in, and/or the power storage unit may be easily replaced if it is faulty. This is not essential, however, and in other embodiments the power storage unit 110 may not be (easily) removable.

The interface 120 is configured to electrically connect to the at least one EM brake of the C-arm system for supplying power to the at least one EM brake when the interface is electrically powered. The interface 120 can thus be understood as an electrical interface capable of supplying power to the EM brake(s) of a C-arm system. In this embodiment, the interface is configured to electrically connect exclusively to the at least one EM brake of the C-arm system. In this way, power from the power storage unit is directed exclusively to the EM brake(s) such that energy usage for the purpose of re-positioning the C-arm system is more efficient. For example, by not also providing power to the X-ray system of the C-arm system, when it is only the EM brake(s) which require power for the repositioning of the C-arm, power usage is made more efficient. This is not essential, however, and in other embodiments, the interface may be configured to electrically connect to any number of components of the C-arm system.

In other words, in this embodiment, the C-arm system comprises at least one motor to drive a movement of the C-arm, a radiation source and/or a detector; and the interface is configured to not electrically connect with any motor, radiation source, or detector of the C-arm system. In this way, power from the power storage unit is reserved for the EM brake(s) of the C-arm system and not wasted on any motors, radiation sources or detectors, even if the C-arm system comprises these components.

The switch 130 is operable between a first configuration and a second configuration, wherein in the first configuration, the switch is configured to permit power supply from the power storage unit to the interface, and in the second configuration, the switch is configured to not permit power supply from the power storage unit to the interface. The switch 130 is configured such that a user is able to interact with it to switch it between the first and second configurations respectively. In some embodiments, the switch 130 is also configured such that a computer or processor is able to interact with it to switch it between the first and second configurations respectively.

In this embodiment, the switch 130 is configured not to be in the first configuration if the EM brake(s) of the C-arm are already being supplied with power from another power source. In this way, the supply of power from the power storage unit 110 can be prevented when it is not needed. For instance, if the C-arm is already connected to mains power, then there is no need for the EM brake(s) to also be supplied with power from the power storage unit 110. Energy efficiency can thus be increased. This is not essential, however, and in other embodiments, the switch 130 may be configured to be able to be put in the first configuration regardless of context.

In this embodiment, the power unit 100 is configured to connect to a key-panel of the C-arm system. In this way, the power unit 100 can be provided in a location that is convenient for a user and which also allows the interface 120 to connect to the electric circuity of the C-arm system. The key-panel can then essentially act as a physical switch and/or button or set of physical switches and/or buttons. This therefore makes efficient use of the physical hardware available. Of course, this feature is only possible if the C-arm system comprises a key-panel, which most, if not all, do. This feature is not essential, however, and in other embodiments, the power unit may be configured to connect in any suitable way to the C-arm system.

In some embodiments, the power unit 100 can further comprise a force detection unit configured to detect a force on the C-arm (i.e., someone trying to manually override the EM brake(s) of the C-arm). In response to the detected force being above some predetermined threshold, the force detection unit can be configured to put the switch 130 in the first configuration. In other words, in response to someone trying to manually override the EM brake(s) of the C-arm, i.e., to manually reposition it while the EM brake(s) are active, the power unit 100 can be configured to automatically supply power to the EM brake(s) such that the C-arm can be repositioned more easily.

In summary, current mobile X-ray imaging systems are getting more and more features added to them for ease of operation and more reliable/effective diagnoses. Motorized movement of a C-arm is one of these features which facilitates precise and effortless movement of the C-arm before/during exposure. This motorized movement feature replaces normal mechanical brakes with electro-mechanical (EM) brakes. To comply with fail safe mechanisms, EM brakes only get released when the system is powered-up. When power is not supplied, or when the system is powered down, the EM brakes remain engaged, with motorized movement prevented and manual movement very difficult. The present invention therefore allows for power to be supplied to the EM brakes (and/or motors) even when the system is in off mode or not supplied with power, and movement of the C-arm in a number of axes (e.g., 3-5) during these times is therefore made possible.

Referring now to Fig. 2, there is depicted a block diagram of a power unit 200 for a C-arm system comprising at least one electro-magnetic (EM) brake for locking movement of a C-arm when not supplied with power according to a proposed embodiment.

The power unit 200 comprises a power storage unit 210, a charging unit 215, an interface 220, a switch 230, and a timer 235.

The power storage unit 210 in this embodiment is connected to a charging unit 215 which is configured to facilitate charging of the power storage unit 210 within the power unit 200, i.e., without having to be removed. In this way, the power storage unit 210 does not need to be removed from the power unit 200 in order for the power storage unit to be recharged and/or for the power unit to be supplied with more power. Of course, in this embodiment, the power storage unit 210 may be removable or non-removable.

In this embodiment, the interface 220 is configured to electrically connect exclusively to the at least one EM brake and at least one motor of the C-arm system. In this way, power can also be provided to motors of the C-arm system such that positioning of the C-arm can be made easier (i.e., by using the motors), while still not providing power to other extraneous components of the C-arm system, such as, for example, its X-ray system. Of course, this feature requires that the C-arm system comprises at least one motor to drive a movement of the C-arm, which many do.

In this embodiment, the switch 230 comprises a physical switch and/or a button. In this way, a user can easily interact with the switch 230 and easily supply power from the power storage unit 210 to the EM brake(s) of the C-arm system when desired. Of course, in other embodiments, the switch 230 can comprise any other suitable physical (or virtual) element that allows interaction, i.e., allows a user to put the switch in the first or second configuration.

In some embodiments, the switch 230 may be operable between the first configuration and the second configuration after a press of the physical switch and/or button of at least five seconds. In this way, false operations of the switch/button can be reduced and thus energy wastage prevented. In other embodiments, the exact time for which the switch needs to be pressed can take other values, for example, two seconds, three seconds, four seconds, six seconds, seven seconds, eight seconds, nine seconds, or ten seconds. In other embodiments, the switch 230 does not need to be pressed for a minimum duration, but rather merely interacted with for any length of time.

In this embodiment, the switch 230 comprises an indicator 232 configured to indicate to a user whether the switch is in the first configuration or the second configuration. This indicator 232 can thus take any suitable form for this purpose. For example, the indicator 232 can comprise a light source configured to emit light in the first configuration and to not emit light in the second configuration. In this way, a user can be supplied with an easy-to-understand visual indication of whether the power unit 200 is currently supplying power to the EM brake(s) of the C-arm system, i.e., which configuration the switch 230 is in. In other embodiments, the indicator 232 can comprise a light source configured inversely to above, i.e., to emit light in the second configuration and to not emit light in the first configuration. This would also provide a user with a visual indication. In other embodiments, the indicator 232 can comprise light sources of (at least) two different colors, such that one color light can be emitted in the first configuration and a second (different) color light can be emitted in the second configuration.

In this embodiment, the switch 230 is configured not to be in the first configuration if the C-arm system is in use. In this way, supply of power from the power storage unit can be prevented when it is not needed.

The timer 235 is configured to put the switch 230 in the second configuration after a predetermined time period of the switch being in the first configuration. In this way, for instance, power can automatically stop being supplied from the power storage unit 210 to the EM brake(s) after, for example, two minutes. This prevents energy waste in cases in which, for example, a user forgets to turn the power supply from the power storage unit 210 back off after they have adjusted the position of the C-arm. For example, two minutes should be long enough for re-positioning of the C-arm to any position, and if it is not, the user may subsequently put the switch back in the first configuration again. The predetermined time period can take any suitable value and, in some embodiments, can be adjusted by a user.

In this embodiment, the timer is programmable by a user or by the C-arm system for adjusting of the predetermined time period. In this way, the timer can be adjusted for increased efficacy and/or efficiency.

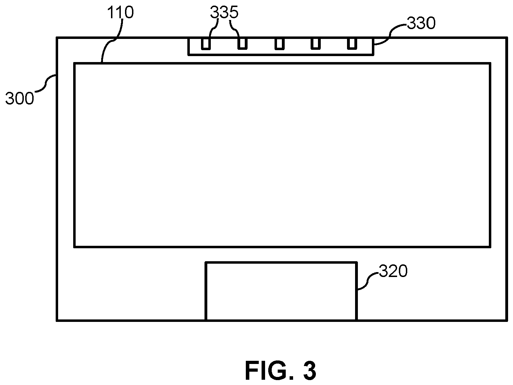

Referring now to Fig. 3, there is depicted a block diagram of a power unit 300 for a C-arm system comprising at least one electro-magnetic (EM) brake for locking movement of a C-arm when not supplied with power according to a proposed embodiment.

The power unit 300 comprises a power storage unit 110, an interface 320, and a switch 330. The power storage unit 110 is much the same as has been described in relation to the power unit 100 of Fig. 1.

In this embodiment, the interface 320 is configured to independently electrically connect to individual EM brakes of the C-arm system for supplying power to each EM brake respectively. In other words, the interface 320 is configured to be able to independently supply power to each individual EM brake of the C-arm system. The switch 330 correspondingly comprises a plurality of sub-switches 335 operable between first and second sub-configurations respectively, wherein in a first sub-configuration, each sub-switch 335 is configured to facilitate electrical communication between the power storage unit and an individual EM brake of the C-arm system respectively, and in a second sub-configuration, each sub-switch 335 is configured to not permit power supply from the power storage unit and an individual EM brake of the C-arm system respectively. For example, the user can interact with each sub-switch 335 independently and therefore, in embodiments, the sub-switches can take any suitable form, for instance, an array of physical (or virtual) switches or buttons. Of course, this feature requires that the C-arm system comprises a plurality of EM brakes, which many do.

In this way, specific EM brakes of the C-arm system can be targeted for deactivation/activation. For instance, movement of the C-arm may only be desired in one particular axis, and therefore, power only needs to be supplied to one EM brake (set) for the desired movement to be achieved. By not automatically supplying power to all EM brakes, but rather allowing users to specify which brakes they want to supply power to, energy efficiency can therefore be improved.

In some embodiments, an individual EM brake can be understood as an individual EM brake set or pair. For instance, when multiple EM brakes are configured for preventing movement of the C-arm in the same axis, they may be considered a set (or pair) of EM brakes. The EM brake set or pair then essentially acts as an individual EM brake and can be understood as such. In this way, a user can essentially select individual movement and/or rotation axes of the C-arm to `activate'.

In some embodiments, two or more sub-switches 335 can be operated simultaneously. In this way, a user can turn multiple EM brakes (or pairs/sets of EM brakes) on or off simultaneously, for instance, from a single command or button press.

In typical C-arm systems, positioning of the C-arm is only possible when the C-arm system is completely up and running. So, in cases where positioning needs to be done quickly and without powering up the entire system, the present invention (the power unit) can be used to facilitate this. Essentially, when the C-arm system is off or not connected to mains power, positioning of the C-arm can be achieved using a battery pack (power storage unit) via a quick actuation key (switch) which releases the EM brake(s) via an interface with the C-arm system. Once the EM locks are disabled, manual positioning of the C-arm can be done. In other words, there is no need to connect the C-arm system to mains power (or to also power a C-arm stand of the C-arm system). This is estimated to reduce the system set-up time by 60-80% as well as making it more user friendly before and/or during the system set-up.

It should be noted that in any of the herein disclosed embodiments (for example, power units 100, 200, 300), the C-arm of the C-arm system for which the power unit is for is configured to translate and/or rotate in at least five axes. In other embodiments, however, the C-arm of the C-arm system can be configured to translate and/or rotate in any number of suitable axes, for instance, any number from one to ten.

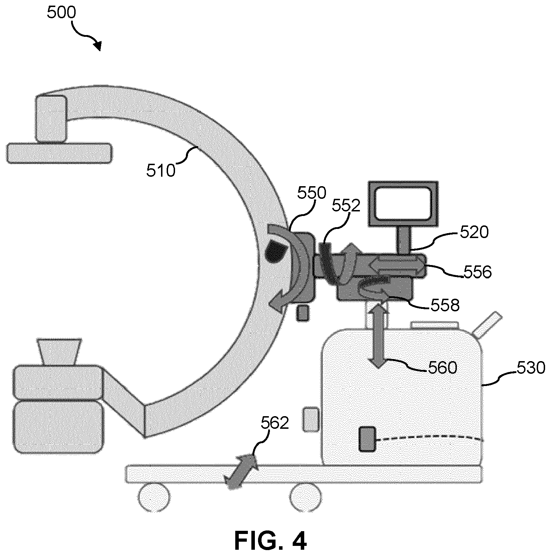

Referring now to Fig. 4, there is depicted an illustration of atypical C-arm system 500 and its axes of motion.

The C-arm system 500 comprises a C-arm 510, a C-arm suspension 520, and a C-arm stand base 530. The C-arm 510 of this C-arm system 500 is configured to move in five axes (i.e., translate and/or rotate) as well as the entire C-arm system 500 able to be transported as shown by arrow 562.

The height of the C-arm 510 is able to be adjusted along axis 560. The C-arm 510 can also be panned as shown by arrow 558. The position of the C-arm 510 can also be adjusted along axis 556, i.e., longitudinally. The propeller of the C-arm 510 can also be adjusted, i.e., the C-arm can be rotated as shown by arrow 552. The angulation of the C-arm 510 can also be adjusted, i.e., the C-arm can be rotated as shown by arrow 550. Any of these motions can be facilitated via motors and/or manually.

Further, although not illustrated in the Figure, each axis of movement for the C-arm system 500, i.e., axes 550, 552, 556, 558, and 560, typically has its own respective EM brake (or pair/set of EM brakes) for preventing movement in each axis respectively.

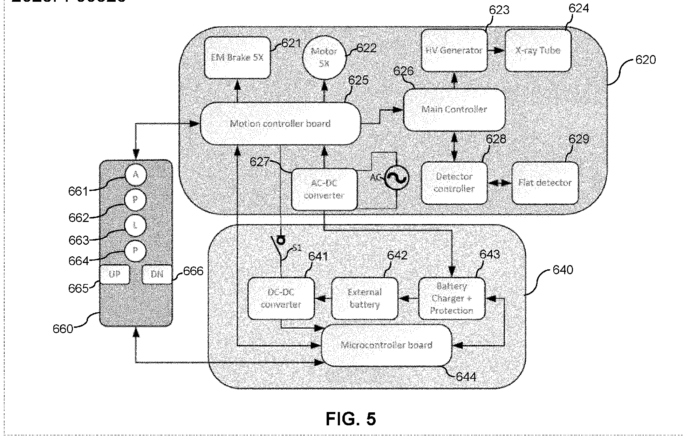

Referring now to Fig. 5, there is depicted a circuit diagram of a C-arm system 620 comprising a power unit 640 according to a proposed embodiment.

The C-arm system assembly 620 comprises five EM brakes 621; five motors 622 (for each of the EM brakes respectively); a high voltage (HV) generator 623; an X-ray tube 624; a motion controller board 625; a main controller 626; an AC-DC converter 627; a detector controller 628; and a flat detector 629.

During normal operation, when a mains supply is available to the C-arm system assembly 620, the motion controller board 625 will be powered up with the AC-DC converter 627 and will instruct motion of the C-arm (via motors 622) as required or directed. For the instructed motion to happen, first the appropriate EM brake(s) 621 needs to be released.

The power unit 640 comprises a switch S1 (which can be understood as akin to switch 130 of power unit 100, with the first configuration being closed and the second configuration being open); a DC-DC converter 641; an external battery 642 (which can be understood as akin to power storage unit 110, 210, 310 of power unit 100, 200, 300); a battery charger 643 (that also functions as protection and can be understood as akin to charging unit 215 of power unit 200); and a microcontroller board 644. Any electrical connections between the power unit 640 and the C-arm system assembly 620 can be understood as the interface of the power unit.

There is also provided a key panel 660 which can function as a physical switch of the power unit 640 comprising physical sub-switches, 661-666. In some embodiments, there may be provided two key panels 660, one for left and one for right. Sub-switch 661 is for providing power to the angulation EM brake(s); sub-switch 662 is for providing power to the propeller EM brake(s); sub-switch 663 is for providing power to the longitudinal EM brake(s); sub-switch 664 is for providing power to the panning EM brake(s); sub-switches 665 and 666 are for providing power to the raising and lowering height EM brake(s) respectively. Each of the sub-switches can also be for providing power to respective motors as well.

For example, for the ease of surgeons/operators, EM brake release keys are typically already provided on the left and right sides of a C-stand. These can function as the switch (or sub-switches) of the power unit 640. By pressing the EM brake keys, EM brakes locking respective axes of motions can be released, and a user can then perform adjustment of the C-arm as required.

Further, the circuity can be such that the if C-arm system assembly 620 is already being provided with mains power, then the power unit 640 will be bypassed. Once a switch is pressed (long-pressed), it can thus activate the motion controller board 625 only to perform the EM brake(s) release/engage and (optionally) activation of motors. The controller 625 can monitor power supply availability and go into sleep mode until power to the C-arm system fails or it has been off for a considerable amount of time, and there is a (long) press of one of the sub-switches on the key panel 660. The use of a long press can help prevent false triggering of the power unit 640 due to a mistaken press or due to a failure in the key panel 660, for example.

As soon as a (sub-)switch of the key panel 660 has been (long-)pressed, the microcontroller board 644 activates and will check mains power availability. If mains power supply is available, it will not perform any action. If mains supply is not available, it will close the S1 switch to facilitate electrical communication with the motion control board 625 of the C-arm system 620. After the motion control board 625 is booted, it will check for mains power supply and if not available, then it will go into 'battery backup mode'.

Battery backup mode will activate only the key panel 660 and the EM brakes 621 (and potentially motors 622 too). Once the key panel 660 is activated, a backlight LED can be turned on, indicating that the key panel 660 is active. When the key panel 660 is active, a user can perform EM brake release and engage in C-arm re-positioning without a mains power supply.

An inbuilt timer can then disconnect the switch S1 after a predetermined period of time and the microcontroller 644 will again go into 'sleep mode'. Considering the fact that a small external battery 642 is getting used for this operation, the use of a timer to de-activate the EM brakes after a predetermined time (such as, 1-2 minutes) can reduce continuous power drain from the battery 642. If required, the process can be started again for more adjustment of the C-arm's position.

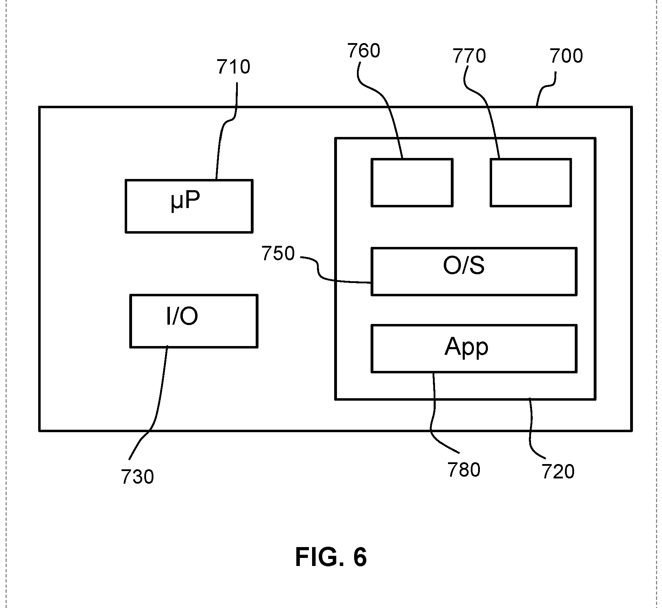

Fig. 6 illustrates an example of a computer 700 within which one or more parts of an embodiment may be employed. Various operations discussed above may utilize the capabilities of the computer 700. In this regard, it is to be understood that system functional blocks can run on a single computer or may be distributed over several computers and locations (e.g. connected via internet).

The computer 700 includes, but is not limited to, PCs, workstations, laptops, PDAs, palm devices, servers, storages, and the like. Generally, in terms of hardware architecture, the computer 700 may include one or more processors 710, memory 720 and one or more I/O devices 730 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 710 is a hardware device for executing software that can be stored in the memory 720. The processor 710 can be virtually any custom made or commercially available processor, a central processing unit (CPU), a digital signal processor (DSP), or an auxiliary processor among several processors associated with the computer 700, and the processor 710 may be a semiconductor based microprocessor (in the form of a microchip) or a microprocessor.

The memory 720 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 720 may incorporate electronic, magnetic, optical, and/or other types of storage media. Note that the memory 720 can have a distributed architecture, where various components are situated remote from one another, but can be accessed by the processor 710.

The software in the memory 720 may include one or more separate programs, each of which comprises an ordered listing of executable instructions for implementing logical functions. The software in the memory 720 includes a suitable operating system (O/S) 750, compiler 760, source code 770, and one or more applications 780 in accordance with exemplary embodiments. As illustrated, the application 780 comprises numerous functional components for implementing the features and operations of the exemplary embodiments. The application 780 of the computer 700 may represent various applications, computational units, logic, functional units, processes, operations, virtual entities, and/or modules in accordance with exemplary embodiments, but the application 780 is not meant to be a limitation.

The operating system 750 controls the execution of other computer programs, and provides scheduling, input-output control, file and data management, memory management, and communication control and related services. It is contemplated by the inventors that the application 780 for implementing exemplary embodiments may be applicable on all commercially available operating systems.

Application 780 may be a source program, executable program (object code), script, or any other entity comprising a set of instructions to be performed. When a source program, then the program is usually translated via a compiler (such as the compiler 760), assembler, interpreter, or the like, which may or may not be included within the memory 720, so as to operate properly in connection with the O/S 750. Furthermore, the application 780 can be written as an object oriented programming language, which has classes of data and methods, or a procedure programming language, which has routines, subroutines, and/or functions, for example but not limited to, C, C++, C#, Pascal, Python, BASIC, API calls, HTML, XHTML, XML, ASP scripts, JavaScript, FORTRAN, COBOL, Perl, Java, ADA, .NET, and the like.

The I/O devices 730 may include input devices such as, for example but not limited to, a mouse, keyboard, scanner, microphone, camera, etc. Furthermore, the I/O devices 730 may also include output devices, for example but not limited to a printer, display, etc. Finally, the I/O devices 730 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 730 also include components for communicating over various networks, such as the Internet or intranet.

If the computer 700 is a PC, workstation, intelligent device or the like, the software in the memory 720 may further include a basic input output system (BIOS) (omitted for simplicity). The BIOS is a set of essential software routines that initialize and test hardware at start-up, start the O/S 750, and support the transfer of data among the hardware devices. The BIOS is stored in some type of read-only-memory, such as ROM, PROM, EPROM, EEPROM or the like, so that the BIOS can be executed when the computer 700 is activated.

When the computer 700 is in operation, the processor 710 is configured to execute software stored within the memory 720, to communicate data to and from the memory 720, and to generally control operations of the computer 700 pursuant to the software. The application 780 and the O/S 750 are read, in whole or in part, by the processor 710, perhaps buffered within the processor 710, and then executed.

When the application 780 is implemented in software it should be noted that the application 780 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 780 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The systems of Figs. 1-3 and 5 may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagrams of Fig. 7 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the Figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions, the architecture, functionality, and operation of possible implementations of systems, methods, and computer program products according to various embodiments of the present invention.

## Claims

1. A power unit (100) for a C-arm system comprising a least one electro-magnetic (EM) brake for locking movement of a C-arm when not supplied with power, the power unit comprising:
a power storage unit (110);
an interface (120) configured to electrically connect to the at least one EM brake of the C-arm system for supplying power to the at least one EM brake when the interface is electrically powered; and
a switch (130) operable between a first configuration and a second configuration, wherein in the first configuration, the switch is configured to permit power supply from the power storage unit to the interface, and in the second configuration, the switch is configured to not permit power supply from the power storage unit to the interface.

2. The power unit claim 1, wherein the C-arm system comprises at least one motor to drive a movement of the C-arm, a radiation source and/or a detector, and wherein the interface (120) is configured to not electrically connect with any motor, radiation source, or detector.

3. The power unit of claim 1 or 2, wherein the interface (120) is configured to electrically connect exclusively to the at least one EM brake of the C-arm system.

4. The power unit of claim 1, wherein the C-arm system comprises at least one motor to drive a movement of the C-arm, and wherein the interface (220) is configured to electrically connect exclusively to the at least one EM brake and the at least one motor of the C-arm system.

5. The power unit of any prior claim, wherein the power unit further comprises a charging unit (215) configured to facilitate charging of the power storage unit within the power unit.

6. The power unit of any prior claim, wherein the power storage unit (110) is removable.

7. The power unit of any prior claim, wherein the power unit further comprises a timer (235) configured to put the switch in the second configuration after a predetermined time period of the switch being in the first configuration, and optionally, wherein the timer is programmable by a user or by the C-arm system for adjusting the predetermined time period.

8. The power unit of any prior claim, wherein the switch (130) is configured not to be in the first configuration if the at least one EM brake of the C-arm is already being supplied with power from another power source.

9. The power unit of any prior claim, wherein the switch (130) is configured not to be in the first configuration if the C-arm system is in use.

10. The power unit of any prior claim, wherein the switch comprises a physical switch and/or a button (230).

11. The power unit of any prior claim, wherein the switch comprises an indicator (232) configured to indicate to a user whether the switch is in the first configuration or the second configuration.

12. The power unit of any prior claim, wherein:
the C-arm system comprises a plurality of EM brakes;
wherein the interface (320) is configured to independently electrically connect to individual EM brakes of the C-arm system for supplying power to each EM brake respectively; and
wherein the switch comprises a plurality of sub-switches (335) operable between first and second sub-configurations respectively, wherein in a first sub-configuration, each sub-switch is configured to facilitate electrical communication between the power storage unit and an individual EM brake of the C-arm system respectively, and in a second sub-configuration, each sub-switch is configured to not permit power supply from the power storage unit and an individual EM brake of the C-arm system respectively.

13. The power unit of claim 12, wherein two or more sub-switches can be operated simultaneously.

14. The power unit of any prior claim, wherein the power unit is configured to connect to a key-panel of the C-arm system.

15. The power unit of any prior claim, wherein the C-arm of the C-arm system is configured to translate and/or rotate in at least five axes.
